# EUROPEAN PATENT APPLICATION

(11) **EP 3 819 906 A1**
(43) Date of publication of application: **12.05.2021**
(21) Application number: 19830867.8
(22) Date of filing: 26.06.2019
(51) Int. Cl.: G16B 20/20, C12N 15/10, C12Q 1/6869, G16B 30/10

(54) **INFORMATION PROCESSING SYSTEM, MUTATION DETECTION SYSTEM, STORAGE MEDIUM, AND INFORMATION PROCESSING METHOD**

(30) Priority: 03.07.2018 JP 2018126455
(71) Applicant: NEC Corporation, 108-8001 Tokyo (JP)
(72) Inventor: HAGIWARA, Hisashi, Tokyo 108-8001 (JP); MISHINA, Yoshinori, Tokyo 108-8001 (JP); YAMAMOTO, Hidenobu, Tokyo 108-8001 (JP); SUGA, Yuko, Tokyo 108-8001 (JP)
(74) Representative: Betten & Resch
(86) International application number: PCT/JP2019/025290
(87) International publication number: WO 2020/008968

(57) **Abstract**

Provided is an information processing system including: a functionality prediction result acquisition unit configured to acquire a result of predicting a functionality of a test target gene in a sequence of a test genome, the test target gene having a sequence different from a reference genome; and a determination unit configured to determine an introduction of an artificial mutation based on the result acquired by the functionality prediction result acquisition unit.

## Description

### [Technical Field]

The example embodiments relate to an information processing system, a mutation detection system, a storage medium, and an information processing method.

### [Background Art]

In Patent Literature 1, there is described a method of detecting presence of deoxyribonucleic acid (DNA) corresponding to soybean event MON87705 in a sample. Further, in Patent Literature 2, there is described a genome editing method including a step of introducing, into a cell or a non-human organism, for example, at least one selected from the group consisting of a guide ribonucleic acid (RNA) 1 targeting any site of genomic DNA and an expression cassette thereof. Moreover, in Patent Literature 3, there is described a method of modifying a targeted site of double-stranded DNA.

### [Citation List]

### [Patent Literature]

PTL 1: Japanese Patent Translation Publication No. 2012-503989
PTL 2: Japanese Patent Application Laid-open No. 2018-011525
PTL 3: Japanese Patent No. 6206893

### [Summary]

### [Technical Problem]

In the method described in Patent Literature 1, an unidentified artificial mutation site cannot be detected. Further, in Patent Literatures 2 and 3, a method of detecting an artificial mutation site is not described.

In view of the above-mentioned problems, an example object of the example embodiments is to provide an information processing system, a mutation detection system, a storage medium, and an information processing method which enable an unidentified artificial mutation site in a nucleic acid sequence to be detected.

### [Solution to Problem]

According to one example aspect of the embodiments, there is provided an information processing system including: a functionality prediction result acquisition unit configured to acquire a result of predicting a functionality of a test target gene in a sequence of a test genome, the test target gene having a sequence different from a reference genome; and a determination unit configured to determine an introduction of an artificial mutation based on the result acquired by the functionality prediction result acquisition unit.

According to another example aspect of the embodiments, there is provided a mutation detection system including: a genome purification unit configured to extract and purify a genome from a cell or a virus; a genome sequence determination unit configured to determine a sequence of the genome obtained by the genome purification unit; and the information processing system described above.

According to still another example aspect of the embodiments, there is provided a storage medium having stored thereon an information processing program for causing a computer to: acquire a result of predicting a functionality of a sequence of a test target gene in a sequence of a test genome, the sequence of the test target gene having a sequence different from a reference genome; and determine an introduction of an artificial mutation based on the result of predicting the functionality.

According to yet another example aspect of the embodiments, there is provided an information processing method including: a functionality prediction result acquisition step of acquiring a result of predicting a functionality of a test target gene in a sequence of a test genome, the test target gene having a sequence different from a reference genome; and a step of determining an introduction of an artificial mutation based on the result acquired in the functionality prediction result acquisition step.

### [Advantageous Effects]

According to the example embodiments, it is possible to provide the information processing system, the mutation detection system, the storage medium, and the information processing method which enable the unidentified artificial mutation site in the nucleic acid sequence to be detected.

### [Brief Description of Drawings]

[Fig. 1]

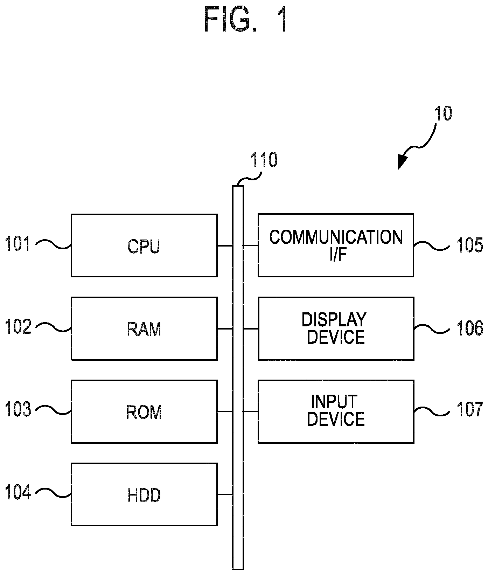

   Fig. 1 is a block diagram for illustrating a hardware configuration example of an information processing system according to a first example embodiment.
[Fig. 2]

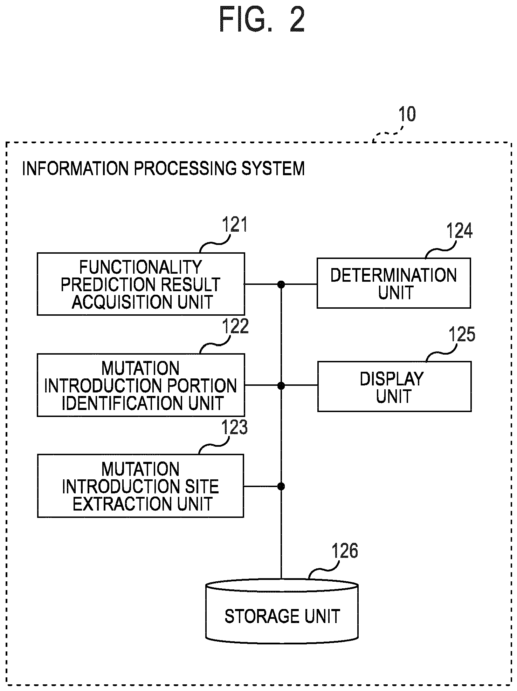

   Fig. 2 is a functional block diagram of the information processing system according to the first example embodiment.
[Fig. 3]

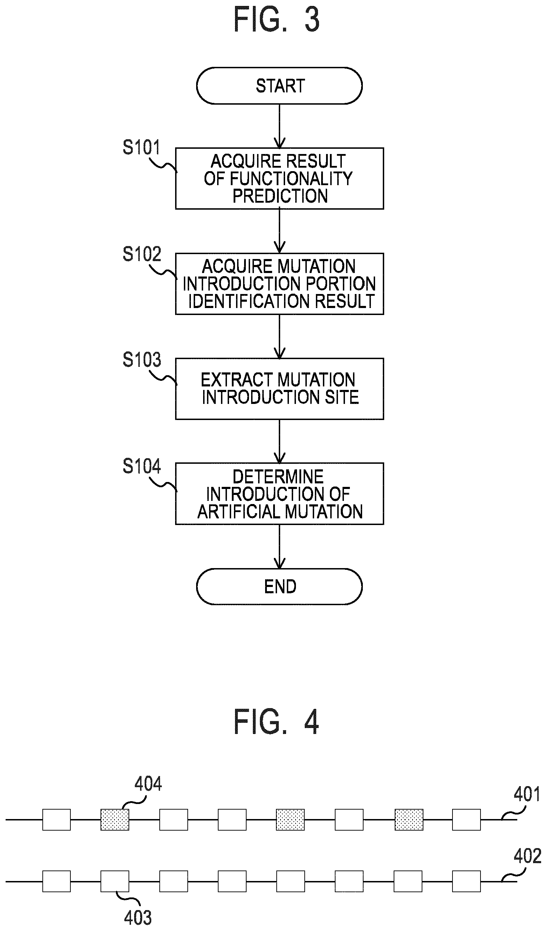

   Fig. 3 is a flowchart for illustrating an outline of processing to be performed by the information processing system according to the first example embodiment.
[Fig. 4]
   Fig. 4 is a schematic diagram for illustrating a comparative analysis.
[Fig. 5]

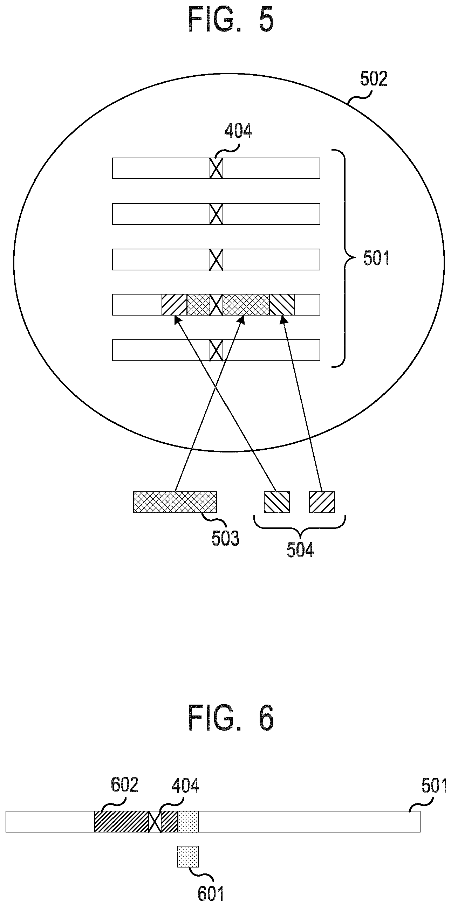

   Fig. 5 is a schematic diagram for illustrating selection of a unique sequence portion including a part or all of a region including a test target gene.
[Fig. 6]
   Fig. 6 is a schematic diagram for illustrating an alignment for identifying a mutation introduction portion.
[Fig. 7]

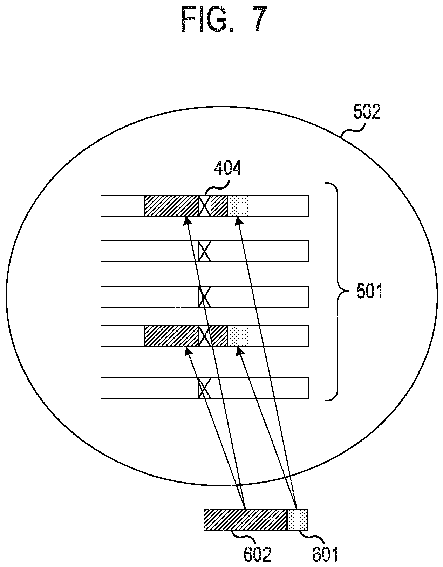

   Fig. 7 is a schematic diagram for illustrating extraction of a mutation introduction site which has a sequence different from a reference genome and includes a PAM sequence and a target sequence from a sequence of a test genome.
[Fig. 8]

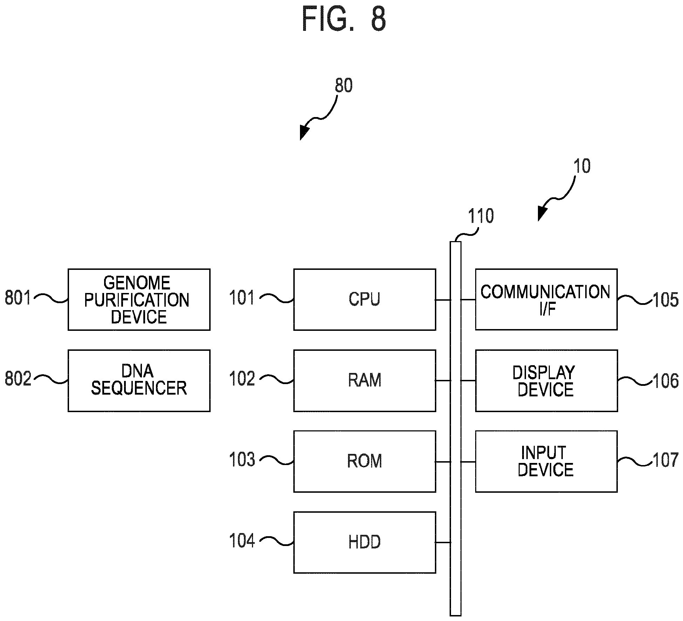

   Fig. 8 is a block diagram for illustrating a hardware configuration example of a mutation detection system according to a second example embodiment.
[Fig. 9]

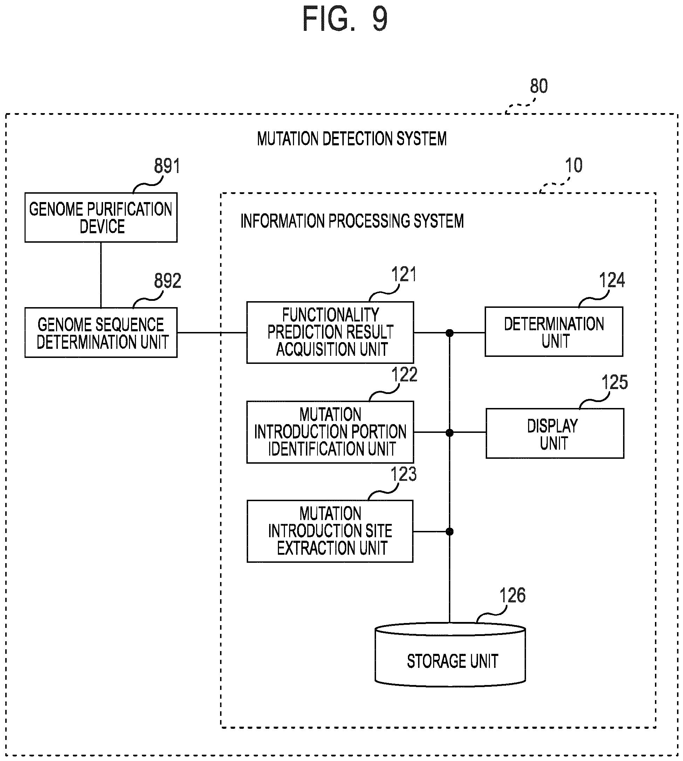

   Fig. 9 is a functional block diagram of the mutation detection system according to the second example embodiment.
[Fig. 10]

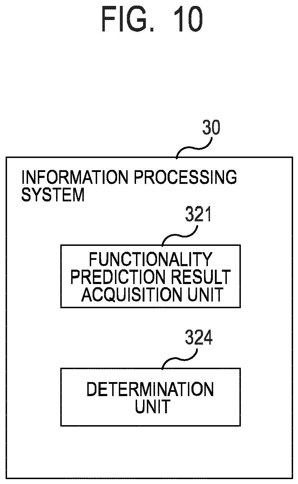

   Fig. 10 is a functional block diagram of an information processing system according to a third example embodiment.

### [Description of Embodiments]

Example embodiments are now described with reference to the drawings. Like elements or corresponding elements are denoted by the same reference numerals in the drawings, and description thereof may be omitted or simplified.

### [First Example Embodiment]

Fig. 1 is a block diagram for illustrating a hardware configuration example of an information processing system 10 according to this example embodiment. The information processing system 10 can be, for example, an artificial mutation site detection device. Further, the information processing system 10 may be a comparison information processing system. The information processing system 10 has functions of a computer. For example, the information processing system 10 may be integrally configured with a desktop personal computer (PC), a laptop PC, a tablet PC, a smartphone, or the like. The information processing system 10 has a function of detecting an unidentified artificial mutation site in a nucleic acid sequence. The information processing system 10 can detect an artificial mutation site by determining that an artificial mutation has been introduced based on a result of predicting a functionality of a test target gene having a sequence different from a reference genome in the sequence of a test genome.

The information processing system 10 can be applied in, for example, detection of an artificial mutation site in the genome of a plant edited for the purpose of producing an illegal drug, detection of an artificial mutation site in the genome in a tissue in which a mutation has been artificially introduced for the purpose of muscle building, detection of an artificial mutation site for the purpose of modifying an individual identification region in human tissue, and detection of an artificial mutation site introduced into, for example, brain tissue for the purpose of manufacturing a biological weapon.

The information processing system 10 includes, in order to implement functions as a computer configured to perform arithmetic operation and storage, a central processing unit (CPU) 101, a random-access memory (RAM) 102, a read-only memory (ROM) 103, and a hard disk drive (HDD) 104. Further, the information processing system 10 includes a communication interface (I/F) 105, a display device 106, and an input device 107. The CPU 101, the RAM 102, the ROM 103, the HDD 104, the communication I/F 105, the display device 106, and the input device 107 are connected to each other via a bus 110. The display device 106 and the input device 107 may be connected to the bus 110 via a drive device (not shown) for driving those devices.

In Fig. 1, the various components forming the information processing system 10 are illustrated as an integrated device, but a part of the functions of those components may be implemented by an external device. For example, the display device 106 and the input device 107 may be external devices different from the components implementing the functions of the computer including the CPU 101, for example.

The CPU 101 is configured to perform predetermined operations in accordance with programs stored in, for example, the ROM 103 and the HDD 104, and also has a function of controlling each component of the information processing system 10. The RAM 102 is built from a volatile storage medium, and is configured to provide a temporary memory area required for the operations of the CPU 101. The ROM 103 is built from a non-volatile storage medium, and is configured to store required information, for example, programs to be used for the operations of the information processing system 10. The HDD 104 is a storage device built from a non-volatile storage medium, and is configured to store genome sequences, for example.

The communication I/F 105 is a communication interface based on a standard, for example, Wi-Fi (trademark) or 4G, and is a module for communicating to and from another device. The display device 106 is, for example, a liquid crystal display or an organic light emitting diode (OLED) display, and is used for displaying moving images, still images, and characters, for example. The input device 107 is, for example, a button, a touch panel, a keyboard, or a pointing device, and is used by a user to operate the information processing system 10. The display device 106 and the input device 107 may be integrally formed as a touch panel.

The hardware configuration illustrated in Fig. 1 is an example, and devices other than the illustrated devices may be added, or a part of the illustrated devices may be omitted. Further, a part of the devices may be substituted with another device having the same function. Moreover, a part of the functions may be provided by another device via a network, and the functions for implementing this example embodiment may be shared and implemented by a plurality of devices. For example, the HDD 104 may be substituted with a solid state drive (SSD) which uses a semiconductor element, for example, a flash memory, or may be substituted with cloud storage.

Fig. 2 is a functional block diagram of the information processing system 10 according to this example embodiment. The information processing system 10 includes a functionality prediction result acquisition unit 121, a mutation introduction portion identification unit 122, a mutation introduction site extraction unit 123, a determination unit 124, a display unit 125, and a storage unit 126.

The CPU 101 implements the functions of the functionality prediction result acquisition unit 121, the mutation introduction portion identification unit 122, the mutation introduction site extraction unit 123, and the determination unit 124 by loading programs stored in the ROM 103, for example, onto the RAM 102 and executing the programs. The processing to be performed by each of those units is described later. The display unit 125 is configured to display information acquired or extracted by the functionality prediction result acquisition unit 121, the mutation introduction portion identification unit 122, the mutation introduction site extraction unit 123, and the determination unit 124. The CPU 101 implements the function of the display unit 125 by controlling the display device 106. The storage unit 126 is configured to store data and the like acquired or extracted by the functionality prediction result acquisition unit 121, the mutation introduction portion identification unit 122, the mutation introduction site extraction unit 123, and the determination unit 124. The CPU 101 implements the function of the storage unit 126 by controlling the HDD 104.

Fig. 3 is a flowchart for illustrating an outline of processing to be performed by the information processing system 10 according to this example embodiment. An outline of the processing to be performed by the information processing system 10 is described with reference to the flowchart of Fig. 3. In the following description, the term "sequence" when used in relation to a genome or a gene may refer to a "base sequence" of the genome or the gene, respectively.

In Step S101 of Fig. 3, the functionality prediction result acquisition unit 121 acquires a result of predicting the functionality of a test target gene having a sequence different from a reference genome in the sequence of a test genome. The test genome is the genome to be tested for presence or absence of a mutation that has been artificially introduced. The reference genome is a genome having a sequence homologous to the test genome before the mutation is artificially introduced. The test target gene is a gene contributing to a trait that is expected to be acquired based on the introduction of the artificial mutation to be detected.

The individual having the test genome is not particularly limited as long as the individual has the genome. Examples thereof may include humans, animals other than humans, plants, yeasts, molds, eubacteria, and viruses.

The reference genome is preferably the genome of a parent strain of the individual having the test genome. Examples of the parent strain include individuals one generation before the individual having the test genome and clones of the individual having the test genome. The genome of an individual one generation before or the genome of a clone of the individual having the test genome has the same sequence as the test genome. That is, the sequence other than the portion of the artificial mutation site is originally the same. Therefore, the load for detecting the artificial mutation site can be reduced, and the possibility of erroneous detection can be reduced.

When the individual having the test genome is a higher organism having a plurality of tissues, the genome sequence of a tissue of the same individual which is different from the tissue having the test genome is also originally the same sequence. Therefore, for the same reason as described above, it is preferred that the reference genome be the genome of a tissue which is of the individual having the test genome and which is different from the tissue having the test genome.

Further, for example, when it is presumed that a part of the same tissue as the tissue having the test genome has been collected and stored before undergoing genome editing, the reference genome can be obtained from the same tissue as the tissue having the test genome before undergoing editing. In this case, the test genome and the reference genome are derived from the same tissue of the same individual, and therefore originally have the same sequence. Therefore, for the same reason as described above, it is preferred that the reference genome be a genome which is obtained from the same tissue as the tissue having the test genome and which is obtained before the test genome.

In the sequence of the test genome, the test target gene having a sequence different from the reference genome can be determined as follows, for example.

The functionality prediction result acquisition unit 121 is configured to, firstly, identify a portion having a sequence different from the reference genome in the sequence of the test genome by performing a comparative analysis between the sequence of the test genome and the sequence of the reference genome. The identification of the portion having a sequence different from the reference genome in the sequence of the test genome by a comparative analysis may be performed by an information processing system different from the information processing system 10.

The sequence of the test genome and the sequence of the reference genome to be used in the comparative analysis may be the sequence of the entire genome, or when the site in which the mutation may be introduced is limited to a specific region, the sequence of the genome of the specific region may be used. It is preferred to acquire the sequence of the entire genome and use the sequence of the entire genome for the comparative analysis because this enables all introduced mutations to be detected without missing any mutations. However, when there is a high certainty that the introduction site of the mutation is limited to a specific region, the genome sequence for only the specific region may be acquired. For example, when it is obvious that the gene involved in acquiring a specific trait is limited to a specific candidate, the genome sequence may be acquired for only the region corresponding to the candidate gene.

The sequence of the test genome and the sequence of the reference genome can be determined by extracting the genome from the cell or, when the individual is a virus, extracting the genome from the virus body, and analyzing the base sequence of the extracted genome. For example, when the individual is a yeast or a mold, for example, the individual may be the cell on which genome extraction is to be performed. Further, for example, when the individual is a human, an animal other than a human, or a plant, a part of a tissue can be collected and used for the cell to be used for genome extraction. At this time, for example, when the individual is a human or an animal other than a human, oral cells or saliva which can be collected painlessly can be used as the tissue to be used for genome extraction.

Extraction of the genome from the cell or the virus body can be performed by carrying out processing appropriate to the individual having the genome. Further, for example, a commercially available kit suitable for the individual having the genome may be used. For example, when extraction from human oral cells or the like is performed, NucleoSpin (trademark) DNA Forensic (manufactured by Takara Bio Inc.) can be used.

The base sequence of the genome obtained by the extraction can be determined by using a commercially available DNA sequencer, for example, a NextSeq series, HiSeq X series (manufactured by Illumina), or PacBio (trademark) RS II/Sequel (trademark) system (manufactured by PacBio) DNA sequencer.

As the reference genome sequence, there may be used a sequence stored in a database which is available to the public by a public organization, for example, the National Human Genome Research Institute (NHGRI), the National Center for Biotechnology Information (NCBI), the DNA Data Bank of Japan (DDBJ) Center, and the Tohoku Medical Megabank Organization. When a sequence is acquired from the database, a sequence having a high homology with the sequence of the reference genome is selected and used. Examples of sequences having high homology with the sequence of the reference genome include genome sequences of individuals belonging to the same species.

The comparative analysis can be performed by using a comparative analysis program, for example, BLASTZ. Fig. 4 is a schematic diagram for illustrating the comparative analysis. The comparative analysis is performed by comparing a sequence 401 of the test genome and a sequence 402 of the reference genome, and identifying a mutation site 404 in a test genome which corresponds to a partial sequence 403 in the reference genome and which has a sequence different from the partial sequence 403 in the reference genome. Specifically, the mutation site 404 identified based on the comparative analysis is a portion in which one or more bases have been deleted, inserted, or substituted when compared with the reference genome.

The term "mutation site" includes artificial mutation sites, natural mutation sites (spontaneous mutation sites), and sites resulting from species diversity. Of those, by setting the reference genome to be the genome of the parent strain or a genome of the tissue of an identical individual, sites resulting from species diversity can be prevented from being included in the unique sequence portion. Therefore, the load for detecting the artificial mutation site can be reduced, and the possibility of erroneous detection can be reduced.

Next, the functionality prediction result acquisition unit 121 sets a sequence including the mutation site and a part of the same sequence in the reference genome adjacent to the mutation site as unique sequence portions, and selects, from among those unique sequence portions, a unique sequence portion including a part or all of the region including the test target gene. The selection of the unique sequence portion including a part or all of the region including the test target gene may be performed by an information processing system different from the information processing system 10. The length of the sequence which is the same as the reference genome included in the unique sequence portion can be freely determined. The sequence portion corresponding to the test target gene in the selected unique sequence portion is the test target gene having a sequence different from the reference genome in the sequence of the test genome.

Specifically, for example, the selection of the unique sequence portion including a part or all of the region including the test target gene can be performed as follows.

Fig. 5 is a schematic diagram for illustrating selection of a unique sequence portion including a part or all of the region including the test target gene. Firstly, the functionality prediction result acquisition unit 121 performs a homology search of a first test control sequence 503 and a second test control sequence 504 by using the sequences of all unique sequence portions 501 as a population 502.

The first test control sequence 503 is a sequence including a part or the entire sequence of the test target gene. The sequence of the test target gene can be acquired from a database available to the public by a public institution, for example, the NHGRI, the NCBI, the DDBJ Center, and the Tohoku Medical Megabank Organization. In order to increase the sensitivity of detection of the artificial mutation site, the first test control sequence 503 is preferably as long as possible, and most preferably the first test control sequence 503 includes the entire sequence of the test target gene.

The second test control sequence 504 is a sequence adjacent to the sequence of the test target gene. The sequence adjacent to the sequence of the test target gene to be used as the second test control sequence 504 may be a sequence upstream from the sequence of the test target gene or a sequence downstream from the sequence of the test target gene. Further, a plurality of second test control sequences 504 may be prepared. For example, as illustrated in Fig. 5, a second test control sequence 504, which is an adjacent sequence on the upstream side of the sequence of the test target gene, and a second test control sequence 504, which is an adjacent sequence on the downstream side of the sequence of the test target gene, may be prepared and used. The length of the second test control sequence can be freely determined, but the length is preferably shorter than the length of the same sequence in the reference genome included in the unique sequence portion 501. When the length of the second test control sequence is shorter than the length of the same sequence in the reference genome included in the unique sequence portion 501, search omissions in the homology search can be suppressed.

Next, the functionality prediction result acquisition unit 121 selects a unique sequence portion 501 having a homology between the sequence of the unique sequence portions 501 found in the homology search and the first test control sequence 503 and/or the second test control sequence 504 higher than a prescribed value. The selected unique sequence portion 501 is a portion including a part or all of the test target gene region. The prescribed value of the homology to be used as a selection criterion can be freely determined in accordance with the test target gene, for example.

When an artificial mutation is introduced into the test target gene and the introduced mutation does not significantly change the sequence of the test target gene, the unique sequence portion including the test target gene into which the mutation has been introduced has a high homology with the first test control sequence, and is selected.

When an artificial mutation is introduced into the test target gene and the introduced mutation significantly changes the sequence of the test target gene, the unique sequence portion including the test target gene into which the mutation has been introduced has a low homology with the first test control sequence. However, the unique sequence portion includes a part of the same sequence as the reference genome adjacent to the sequence different from the reference genome. That is, the unique sequence portion includes a sequence in which a mutation has not been introduced and which is adjacent to the test target gene into which a mutation has been introduced. This sequence is a portion corresponding to the second test control sequence. Therefore, in a case in which the introduced mutation significantly changes the sequence of the test target gene, for example, even when the mutation has caused all of the test target gene to be deleted, the sequence can be selected as a unique sequence portion having a high homology with the second test control sequence.

However, when the mutation site in the unique sequence portion is not included in the portion corresponding to the first test control sequence and is included in the portion corresponding to the second test control sequence, the functionality prediction result acquisition unit 121 does not select that unique sequence portion. This is because such a unique sequence portion is not considered to be the artificial mutation that is the target of detection.

The prediction of the functionality of a test target gene having a sequence different from the reference genome in the sequence of the test genome can be performed in accordance with a criterion determined in advance based on the test target gene to be tested. As used herein, "functionality" refers to the acquisition of a trait expected to have arisen due to the introduction of the artificial mutation.

That is, for example, when the expected trait is acquired as a result of the introduced mutation causing the test target gene to lose a function that the test target gene originally had, a criterion for determining whether or not the mutation causes the test target gene to lose the original function is determined in advance. In particular, when the number of bases which are inserted or deleted on the upstream side (5'-end side) of the test target gene is not a multiple of three, a frame shift occurs in the translation process of gene expression, and as a result, there is a high possibility that the test target gene loses the function that the test target gene originally had. Moreover, mutations in which a stop codon is introduced by base substitution or insertion, particularly on the upstream side (5'-end side) of the test target gene, may also cause immature messenger RNA to be produced in the transcription process of gene expression, and as a result, there is a high possibility that the mutation causes the test target gene to lose the function that the test target gene originally had. In addition, mutations which cause most or all of the test target gene to be deleted can also be a mutation which causes the test target gene to lose the function that the test target gene originally had.

Further, for example, when a test target gene which is not originally present in the test genome is introduced as a mutation and the expected trait is acquired as a result of the function of the test target gene, whether or not the test target gene has been introduced can be used as the determination criterion.

Moreover, for example, when the expected trait is acquired by acquiring a function different from the function that the test target gene originally had as a result of the introduced mutation, a criterion for determining whether or not a function different from the function that the test target gene originally had is acquired is determined in advance.

The criterion to be used to predict the functionality may also be determined by, for example, using a research paper search engine, for example, PubMed, to acquire and refer to academic papers based on keywords relating to the target trait. Further, for example, a program, for example, Jpred, may be used to predict the structure of a peptide (protein) to be translated based on an amino acid sequence read from the base sequence of the test target gene or to refer to the three-dimensional structure of the protein stored in a database, for example, Protein Data Bank (PDB).

In Step S101, the functionality prediction result acquisition unit 121 acquires a result of predicting the functionality in accordance with a certain criterion as described above.

In Step S102, the mutation introduction portion identification unit 122 acquires a result of identifying, in the sequence including the test target gene, a mutation introduction portion including a PAM sequence and a target sequence which are usable in editing using a CRISPR-Cas9 system.

The sequence including the test target gene corresponds to a unique sequence portion selected in the manner described above. The PAM sequence is a protospacer adjacent motif, and the target sequence is a target sequence adjacent to the PAM sequence, which are each used for editing using the CRISPR-Cas9 system.

Fig. 6 is a schematic diagram for illustrating an alignment for identifying a mutation introduction portion. For example, the mutation introduction portion identification unit 122 can identify the mutation introduction portion as follows. Firstly, a PAM sequence 601 is aligned with the selected unique sequence portion 501. Next, the position of the PAM sequence 601 is identified, and the sequence having a specific number of bases adjacent to the PAM sequence 601 on the upstream side is identified as a target sequence 602. The alignment can be performed by pairwise alignment, for example. The identification of the mutation introduction portion may be performed by an information processing system different from the information processing system 10.

Examples of combinations of a bacterial strain derived from Cas9 nuclease used for editing using the CRISPR-Cas9 system and the PAM sequence recognized by each subtype of the Cas9 nuclease include 5'-NGG (Streptococcus pyogenes, type II), 5'-CCN (Sulfolobus solfataricus, type I-A1), 5'-TCN (Sulfolobus solfataricus, type I-A2), 5'-TTC (Haloquadratum walsbyi, type I-B), 5'-AWG (Escherichia coli, type I-E), 5'-CC (Escherichia coli, type I-F), 5'-CC (Pseudomonas aeruginosa, type I-F), 5'-NNAGAA (Streptococcus thermophilus, type II-A), and 5'-NGG (Streptococcus agalactiae, type II-A).

The number of bases in the sequence to be identified as the target sequence is determined in accordance with each subtype of the Cas9 nuclease corresponding to the PAM sequence having an identified position. For example, when the Cas9 nuclease used for editing using the CRISPR-Cas9 system is derived from Streptococcus pyogenes, type II, the number of bases is 19 or 20.

In editing using the CRISPR-Cas9 system, a mutation is introduced to the portion corresponding to the target sequence adjacent to the PAM sequence. Therefore, when a base in the unique sequence portion which is different between the test genome sequence and the reference genome sequence is present in the target sequence, it can be considered that the mutation is a mutation which has been artificially introduced by using the CRISPR-Cas9 system.

In Step S103, when a sequence different from the reference genome in the sequence of the test genome is present in the target sequence in the result acquired by the mutation introduction portion identification unit 122, the mutation introduction site extraction unit 123 extracts a mutation introduction site which has a sequence different from the reference genome and which includes the PAM sequence and the target sequence from the sequence of the test genome.

The mutation introduction site extraction unit 123 can perform the extraction of a mutation introduction site which has a sequence different from the reference genome and which includes the PAM sequence and the target sequence from the sequence of the test genome by, for example, acquiring information on a unique sequence portion selected as follows.

Fig. 7 is a schematic diagram for illustrating extraction of a mutation introduction site which has a sequence different from the reference genome and which includes the PAM sequence and the target sequence from the sequence of the test genome. Firstly, the mutation introduction site extraction unit 123 performs a homology search on combinations of the PAM sequence 601 and the target sequence 602 identified as having a sequence different from the reference genome in the sequence of the test genome by using the sequences of all the unique sequence portions 501 as the population 502. Next, the mutation introduction site extraction unit 123 selects a unique sequence having a homology higher than a prescribed value. The prescribed value can be freely set. The homology search on the combinations of the PAM sequence 601 and the target sequence 602 and the selection of a unique sequence having a higher homology than the prescribed value may be performed by an information processing system different from the information processing system 10.

It is known that, in editing using the CRISPR-Cas9 system, editing may be performed in a non-specific manner on a site different from the target site. Therefore, when an artificial mutation has been introduced into the sequence of the test genome by using the CRISPR-Cas9 system, there is a possibility that a mutation is simultaneously introduced into a sequence other than the sequence of the test target gene. The site into which a mutation has been non-specifically introduced in the test genome has a sequence different from that of the reference genome, and therefore the functionality prediction result acquisition unit 121 identifies the mutation as a unique sequence portion based on the comparative analysis described above.

Further, in editing using the CRISPR-Cas9 system, a PAM sequence and a target sequence are included, and therefore the site into which a mutation has been non-specifically introduced is identified as a unique sequence portion having a high homology in the above-mentioned homology search, and can be selected. That is, when the result extracted by the mutation introduction site extraction unit 123 includes a unique sequence portion having a higher homology than a certain value set as the prescribed value, it can be considered that editing using the CRISPR-Cas9 system has been performed.

In Step S104, the determination unit 124 determines an introduction of an artificial mutation. The determination unit 124 can detect an artificial mutation site by determining that an artificial mutation has been introduced. The determination unit 124 can determine that an artificial mutation has been introduced when, for example, the result extracted by the mutation introduction site extraction unit 123 includes one or more unique sequence portions having a higher homology than a certain value set as the prescribed value.

In this example embodiment, as an example, there is described a case in which the information processing system 10 includes all of the functionality prediction result acquisition unit 121, the mutation introduction portion identification unit 122, and the mutation introduction site extraction unit 123, but the example embodiments is not limited thereto.

For example, the information processing system 10 may not include the mutation introduction site extraction unit 123, and only include the functionality prediction result acquisition unit 121 and the mutation introduction portion identification unit 122. In such a case, the determination unit 124 can determine that an artificial mutation has been introduced when, for example, the result acquired by the mutation introduction portion identification unit 122 indicates that a mutation is present in the target sequence.

Further, for example, the information processing system 10 may not include the mutation introduction portion identification unit 122 and the mutation introduction site extraction unit 123, and only include the functionality prediction result acquisition unit 121. In such a case, the determination unit 124 can determine that an artificial mutation has been introduced when, for example, the result acquired by the functionality prediction result acquisition unit 121 indicates that the test target gene into which the mutation has been introduced is predicted to have functionality. Moreover, the method to be used to introduce the artificial mutation to be detected is not limited to editing using the CRISPR-Cas9 system.

From the viewpoint of increasing the accuracy of the result determined by the determination unit 124, the information processing system 10 preferably includes the mutation introduction portion identification unit 122, and more preferably includes the mutation introduction site extraction unit 123.

### [Second Example Embodiment]

The above-mentioned information processing system 10 can form a mutation detection system together with a genome purification unit and a genome sequence determination unit.

Fig. 8 is a block diagram for illustrating a hardware configuration example of a mutation detection system according to a second example embodiment. A mutation detection system 80 includes a genome purification device 801, a DNA sequencer 802, and the information processing system 10. The configuration of the information processing system 10 is the same as that described above. The hardware configuration illustrated in Fig. 8 is an example, and devices other than the illustrated devices may be added, or a part of the illustrated devices may be omitted. Further, a part of the devices may be substituted with another device having the same function. Moreover, a part of the functions may be provided by another device via a network, and the functions for implementing this example embodiment may be shared and implemented by a plurality of devices.

Fig. 9 is a functional block diagram of the mutation detection system 80 according to the second example embodiment. The genome purification device 801 is configured to implement a function of a genome purification unit 891, and the DNA sequencer 802 is configured to implement a function of a genome sequence determination unit 892.

The genome purification unit 891 is configured to purify the genome from a cell or the individual having the test genome. Further, the genome may be purified from a cell or the individual of the parent strain of the individual having the test genome, or from a cell of tissue of the individual having the test genome. Extraction of the genome from a cell or a virus body can be performed by performing appropriate processing suitable for the individual having the genome.

The genome sequence determination unit 892 is configured to determine a base sequence of the genome purified by the genome purification unit 891. The base sequence to be determined may be the entire base sequence of the genome or the base sequence of a specific region of the genome, but it is preferred to determine the entire base sequence of the genome. The base sequence of the genome can be determined by next-generation sequencing, for example.

The information processing system 10 detects an artificial mutation site by using the base sequence of the genome determined by the genome sequence determination unit 892. The details of the detection of the artificial mutation site in the information processing system 10 are the same as those described above.

### [Third Example Embodiment]

Fig. 10 is a functional block diagram of an information processing system 30 according to a third example embodiment. The information processing system 30 includes a functionality prediction result acquisition unit 321 and a determination unit 324. The functionality prediction result acquisition unit 321 is configured to acquire a result of predicting the functionality of a test target gene having a sequence different from the reference genome in the sequence of the test genome. The determination unit 324 is configured to determine the introduction of an artificial mutation.

According to this example embodiment, there can be provided an information processing system capable of detecting an unidentified artificial mutation site in a nucleic acid sequence.

The above-mentioned example embodiments merely describe specific examples in carrying out the embodiments, and are not to be construed as limiting the technical scope of the embodiments in any way. That is, the example embodiments can be implemented in various forms without departing from the technical idea or the main features of the example embodiments.

The whole or part of the example embodiments disclosed above can be described as, but not limited to, the following supplementary notes.

### (Supplementary note 1)

An information processing system comprising:
a functionality prediction result acquisition unit configured to acquire a result of predicting a functionality of a test target gene in a sequence of a test genome, the test target gene having a sequence different from a reference genome; and
a determination unit configured to determine an introduction of an artificial mutation based on the result acquired by the functionality prediction result acquisition unit.

### (Supplementary note 2)

The information processing system according to claim 1, further comprising a mutation introduction portion identification unit configured to acquire a result of identifying, in the sequence including the test target gene, a mutation introduction portion including a PAM sequence and a target sequence which are usable in editing using a CRISPR-Cas9 system.

### (Supplementary note 3)

The information processing system according to claim 2, further comprising a mutation introduction site extraction unit configured to extract, from the sequence of the test genome, a mutation introduction site which has a sequence different from the reference genome and which includes the PAM sequence and the target sequence when a sequence different from the reference genome in the sequence of the test genome is present in the target sequence in the result acquired by the mutation introduction portion identification unit.

### (Supplementary note 4)

The information processing system according to any one of claims 1 to 3, wherein the reference genome is a genome of a parent strain of an individual having the test genome.

### (Supplementary note 5)

The information processing system according to any one of claims 1 to 3, wherein the reference genome is a genome of a tissue which is of an individual having the test genome and which is different from a tissue having the test genome.

### (Supplementary note 6)

The information processing system according to any one of claims 1 to 3, wherein the reference genome is a genome which is obtained from the same tissue as a tissue having the test genome and which is obtained before the test genome.

### (Supplementary note 7)

A mutation detection system comprising:
a genome purification unit configured to extract and purify a genome from a cell or a virus;
a genome sequence determination unit configured to determine a sequence of the genome obtained by the genome purification unit; and
the information processing system of any one of claims 1 to 6.

### (Supplementary note 8)

A storage medium having stored thereon an information processing program for causing a computer to:
acquire a result of predicting a functionality of a sequence of a test target gene in a sequence of a test genome, the sequence of the test target gene having a sequence different from a reference genome; and
determine an introduction of an artificial mutation based on the result of predicting the functionality.

### (Supplementary note 9)

The storage medium having stored thereon an information processing program according to claim 8, wherein the information processing program further causes the computer to acquire a result of identifying, in the sequence including the test target gene, a mutation introduction portion including a PAM sequence and a target sequence which are usable in editing using a CRISPR-Cas9 system.

### (Supplementary note 10)

The storage medium having stored thereon an information processing program according to claim 9, wherein the information processing program further causes the computer to extract, from the sequence of the test genome, a mutation introduction site which has a sequence different from the reference genome and which includes the PAM sequence and the target sequence when a sequence different from the reference genome in the sequence of the test genome is present in the target sequence in the result of identifying the mutation introduction portion.

### (Supplementary note 11)

An information processing method comprising:
a functionality prediction result acquisition step of acquiring a result of predicting a functionality of a test target gene in a sequence of a test genome, the test target gene having a sequence different from a reference genome; and
a step of determining an introduction of an artificial mutation based on the result acquired in the functionality prediction result acquisition step.

### (Supplementary note 12)

The information processing method according to claim 11, further comprising a mutation introduction portion identification step of acquiring a result of identifying, in the sequence including the test target gene, a mutation introduction portion including a PAM sequence and a target sequence which are usable in editing using a CRISPR-Cas9 system.

### (Supplementary note 13)

The information processing method according to claim 12, further comprising a mutation introduction site extraction step of extracting, from the sequence of the test genome, a mutation introduction site which has a sequence different from the reference genome and which includes the PAM sequence and the target sequence when a sequence different from the reference genome in the sequence of the test genome is present in the target sequence in the result acquired in the mutation introduction portion identification step.

This application is based upon and claims the benefit of priority from Japanese Patent Application No. 2018-126455, filed on July 3, 2018, the disclosure of which is incorporated herein in its entirety by reference.

### [Reference Signs List]

10, 30 information processing system
80 mutation detection system
101 CPU
102 RAM
103 ROM
104 HDD
105 communication I/F
106 display device
107 input device
110 bus
121, 321 functionality prediction result acquisition unit
122 mutation introduction portion identification unit
123 mutation introduction site extraction unit
124, 324 determination unit
125 display unit
126 storage unit
801 genome purification device
802 DNA sequencer
891 genome purification unit
892 genome sequence determination unit

## Claims

1. An information processing system comprising:
a functionality prediction result acquisition unit configured to acquire a result of predicting a functionality of a test target gene in a sequence of a test genome, the test target gene having a sequence different from a reference genome; and
a determination unit configured to determine an introduction of an artificial mutation based on the result acquired by the functionality prediction result acquisition unit.

2. The information processing system according to claim 1, further comprising a mutation introduction portion identification unit configured to acquire a result of identifying, in the sequence including the test target gene, a mutation introduction portion including a PAM sequence and a target sequence which are usable in editing using a CRISPR-Cas9 system.

3. The information processing system according to claim 2, further comprising a mutation introduction site extraction unit configured to extract, from the sequence of the test genome, a mutation introduction site which has a sequence different from the reference genome and which includes the PAM sequence and the target sequence when a sequence different from the reference genome in the sequence of the test genome is present in the target sequence in the result acquired by the mutation introduction portion identification unit.

4. The information processing system according to any one of claims 1 to 3, wherein the reference genome is a genome of a parent strain of an individual having the test genome.

5. The information processing system according to any one of claims 1 to 3, wherein the reference genome is a genome of a tissue which is of an individual having the test genome and which is different from a tissue having the test genome.

6. The information processing system according to any one of claims 1 to 3, wherein the reference genome is a genome which is obtained from the same tissue as a tissue having the test genome and which is obtained before the test genome.

7. A mutation detection system comprising:
a genome purification unit configured to extract and purify a genome from a cell or a virus;
a genome sequence determination unit configured to determine a sequence of the genome obtained by the genome purification unit; and
the information processing system of any one of claims 1 to 6.

8. A storage medium having stored thereon an information processing program for causing a computer to:
acquire a result of predicting a functionality of a sequence of a test target gene in a sequence of a test genome, the sequence of the test target gene having a sequence different from a reference genome; and
determine an introduction of an artificial mutation based on the result of predicting the functionality.

9. The storage medium having stored thereon an information processing program according to claim 8, wherein the information processing program further causes the computer to acquire a result of identifying, in the sequence including the test target gene, a mutation introduction portion including a PAM sequence and a target sequence which are usable in editing using a CRISPR-Cas9 system.

10. The storage medium having stored thereon an information processing program according to claim 9, wherein the information processing program further causes the computer to extract, from the sequence of the test genome, a mutation introduction site which has a sequence different from the reference genome and which includes the PAM sequence and the target sequence when a sequence different from the reference genome in the sequence of the test genome is present in the target sequence in the result of identifying the mutation introduction portion.

11. An information processing method comprising:
a functionality prediction result acquisition step of acquiring a result of predicting a functionality of a test target gene in a sequence of a test genome, the test target gene having a sequence different from a reference genome; and
a step of determining an introduction of an artificial mutation based on the result acquired in the functionality prediction result acquisition step.

12. The information processing method according to claim 11, further comprising a mutation introduction portion identification step of acquiring a result of identifying, in the sequence including the test target gene, a mutation introduction portion including a PAM sequence and a target sequence which are usable in editing using a CRISPR-Cas9 system.

13. The information processing method according to claim 12, further comprising a mutation introduction site extraction step of extracting, from the sequence of the test genome, a mutation introduction site which has a sequence different from the reference genome and which includes the PAM sequence and the target sequence when a sequence different from the reference genome in the sequence of the test genome is present in the target sequence in the result acquired in the mutation introduction portion identification step.
